(19) Europäisches Patentamt / European Patent Office / Office européen des brevets

(11) **EP 2 500 083 A1**

(12) **EUROPEAN PATENT APPLICATION**
published in accordance with Art. 153(4) EPC

(43) Date of publication:
**19.09.2012 Bulletin 2012/38**

(51) Int Cl.:
**B01D 63/04** *(2006.01)* **B01D 63/00** *(2006.01)*
**B01D 71/34** *(2006.01)* **C12M 1/12** *(2006.01)*
**C12M 3/06** *(2006.01)* **C12N 15/09** *(2006.01)*
**C12P 7/56** *(2006.01)*

(21) Application number: **10829942.1**

(22) Date of filing: **10.11.2010**

(86) International application number:
**PCT/JP2010/069969**

(87) International publication number:
**WO 2011/058983 (19.05.2011 Gazette 2011/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2009 JP 2009256778**

(71) Applicant: **Toray Industries, Inc.**
**Tokyo 103-8666 (JP)**

(72) Inventors:
- **TAKEUCHI, Norihiro**
  **Otsu-shi**
  **Shiga 520-8558 (JP)**
- **MINEGISHI, Shin-ichi**
  **Urayasu-shi**
  **Chiba 279-8555 (JP)**
- **CHEON, Jihoon**
  **Otsu-shi**
  **Shiga 520-8558 (JP)**
- **NISHIDA, Makoto**
  **Otsu-shi**
  **Shiga 520-8558 (JP)**
- **MIMITSUKA, Takashi**
  **Kamakura-shi**
  **Kanagawa 248-8555 (JP)**
- **SUZUKI, Hironobu**
  **Otsu-shi**
  **Shiga 520-8558 (JP)**
- **YAMADA, Katsushige**
  **Kamakura-shi**
  **Kanagawa 248-8555 (JP)**
- **SAWAI, Hideki**
  **Kamakura-shi**
  **Kanagawa 248-8555 (JP)**
- **KUMO, Ichiro**
  **Otsu-shi**
  **Shiga 520-8558 (JP)**

(74) Representative: **Hoefer & Partner**
**Pilgersheimer Straße 20**
**81543 München (DE)**

(54) **HOLLOW FIBER MEMBRANE MODULE FOR USE IN PRODUCTION OF CHEMICAL SUBSTANCE, AND PROCESS FOR PRODUCTION OF CHEMICAL SUBSTANCE**

(57) A hollow fiber membrane module for use in production of a chemical substance, which is used in continuous fermentation including filtering a fermentation broth of a microorganism or a cultured cell through a hollow fiber membrane, collecting a chemical substance from a filtrate, retaining a concentrated solution in the fermentation broth or refluxing the concentrated solution, and adding a fermentation raw material to the fermentation broth, wherein a large number of hollow fiber membrane bundles are accommodated in a tubular case, at least one end part of each of the hollow fiber membrane bundles is fixed on the tubular case by a hollow fiber membrane bundling member with an end face of each of the hollow fiber membranes open, and the hollow fiber membrane bundling member is made of a synthetic resin having a hardness retention rate after contact with saturated steam at 121°C for 24 hours of 95% or more. Therefore, a hollow fiber membrane module for use in the production of a chemical substance by a continuous fermentation process capable of maintaining high productivity and treating steam sterilization is provided.

EP 2 500 083 A1

# FIG.4

(CULTURE MEDIUM)

(NEUTRALIZING SOLUTION)

17 18 19 14 13

12

15

(GAS)

16 20 1

**Description**

Field

The present invention relates to a hollow fiber membrane module for use in a production of a chemical substance, which is designed so that the reduction of filtration property by clogging is unlikely to occur in order to increase the concentration of a microorganism involved in fermentation and to achieve high productivity, in a process for production of a chemical substance by a continuous fermentation process which includes filtering a liquid containing the chemical substance from a fermentation broth of a microorganism or a cultured cell through the hollow fiber membrane module while culturing, collecting it, returning a liquid which has not been filtered to the fermentation broth, and adding a fermentation raw material to the fermentation broth.

Background

Fermentation process which is a process for production of a substance with culture of a microorganism or a cultured cell can be broadly classified into (1) a batch fermentation process and a fed-batch fermentation process, and (2) a continuous fermentation process.

The batch fermentation process and the fed-batch fermentation process of the above (1) have advantages of simple equipment and little damage caused by bacteria contamination since culture is completed for a short time. However, the concentration of a chemical substance in a fermentation broth increases over time, and productivity and yield decrease by effects of an osmotic pressure, chemical substance inhibition, or the like. Therefore, it is difficult to stably maintain high yield and high productivity over a long period of time.

Further, the continuous fermentation process of the above (2) is characterized in that high yield and high productivity can be maintained over a long period of time by avoiding accumulation of a target chemical substance in high concentration in a fermenter. As for the continuous fermentation process, a continuous culture process involved in fermentation of L-glutamic acid or L-lysine has been disclosed (see Non Patent Literature 1). However, in this example, while a raw material is continuously supplied to a fermentation broth, a fermentation broth containing a microorganism or a cultured cell is taken out. Thus, the microorganism or the cultured cell in the fermentation broth is diluted, and the improvement of production efficiency is restricted.

In the continuous fermentation process, a process for keeping the concentration of a microorganism or a cultured cell high in a fermentation broth by filtering the microorganism or the cultured cell through a separation membrane and collecting a chemical substance from a filtrate, and at the same time retaining or refluxing the microorganism or the cultured cell in a concentrated liquid in the fermentation broth has been proposed.

For example, a technique of continuous fermentation in a continuous fermentation apparatus using a flat membrane made of an organic macromolecule as the separation membrane has been proposed (Patent Literature 1). However, in the proposed technique, an effective membrane area relative to an installed volume of a flat membrane unit is small, a cost advantage obtained by the production of a target chemical substance through this technique is not sufficient, or the like. Accordingly, it has been an ineffective technique.

In order to solve the problem, a continuous fermentation technique in which a hollow fiber membrane made of an organic macromolecule is used as a separation membrane used in the continuous fermentation apparatus has been proposed (Patent Literature 2). In this technique, a membrane unit can have a large membrane area per unit volume. Therefore, a fermentation production efficiency is much higher as compared with the conventional continuous fermentation.

As a separation membrane module using a hollow fiber membrane, there has been a module in which a large number of hollow fiber membrane bundles are accommodated in a tubular case, both end parts of each of the hollow fiber membrane bundles are fixed on the tubular case by a hollow fiber membrane bundling member with at least one end face of each of hollow fiber membranes open. In addition to this, in order to easily detach blocking matters accumulated inside the hollow fiber membrane bundles and sufficiently develop separation performance, for example, a technique of a hollow fiber membrane module for a water treatment in which one end of each of hollow fiber membranes is not fixed in a case and each of the hollow fiber membranes is singly sealed to remarkably improve discharging property of suspended matters has been disclosed (see Patent Literature 3). However, in the hollow fiber membrane module of this configuration, an operation of singly sealing the end face of each of a large number of hollow fiber membranes is complex, and it takes a long time to perform the operation. Further, when raw water and air for cleaning are supplied, the hollow fiber membranes vibrate hard more than necessary to get entangled, or are broken. Thus, the hollow fiber membranes get damage.

Moreover, a process for sealing a hollow fiber membrane by dividing a lower end of each of hollow fiber membrane bundles on a sealing side into a plurality of small bundles, and adhering each of the small bundles with a resin has been disclosed as a configuration of a hollow fiber membrane module in which the discharging property of suspended matters is good and the operation of sealing a hollow fiber membrane is easy (see Patent Literature 4).

However, it is difficult to use the module using a hollow fiber membrane as a separation membrane module for production of a chemical substance by continuous fermentation as it is.

This is because the production of a chemical substance by continuous fermentation requires culture in such a manner that bacterial contamination is basically prevented. For example, when bacteria are contaminated from the separation membrane module during the filtration of a fermentation broth, the chemical substance is not effectively produced by decrease of fermentation efficiency, foaming in a fermenter, or the like. For this reason, the sterilization of each separation membrane module is required to prevent bacterial contamination. Examples of sterilization methods may include flame sterilization, dry heat sterilization, boiling sterilization, steam sterilization, sterilization by ultraviolet irradiation, sterilization by gamma irradiation, gas sterilization, and the like. However, when a chemical substance is produced in accordance with Patent Literature 2, it should be noted that a separation function is lost by drying a membrane used in the above Literature. For this reason, in order to perform sterilization so as not to lose moisture in the separation membrane, steam sterilization (usually 121°C for 15 to 20 minutes) is a suitable sterilization method. Patent Literature 4 does not disclose a response to a heat treatment under a temperature condition in which a separation membrane module is subjected to steam sterilization. In this case, there is concern that when the separation membrane module is subjected to steam sterilization, thermal degradation of materials occurs, causing a problem of partial damage of the module.

Further, the continuous fermentation process using a separation membrane module requires that the concentration of a microorganism or a cultured cell in a fermentation broth is kept high by filtering the microorganism or the cultured cell through a separation membrane so that the separation membrane in the separation membrane module does not clog, and collecting a chemical substance from a filtrate, and at the same time retaining or refluxing the microorganism or the cultured cell in a concentrated liquid in the fermentation broth. However, Patent Literature 2 does not describe or suggest a design of a separation membrane module for filtration of broth of pure microorganism having a high concentration which sufficiently develops a performance of a hollow fiber separation membrane.

Citation List

Patent Literature

Patent Literature 1: Japanese Patent Application Laid-open No. 2007-252367
Patent Literature 2: Japanese Patent Application Laid-open No. 2008-237101
Patent Literature 3: Japanese Patent Application Laid-open No. H07-60074
Patent Literature 4: Japanese Patent Application Laid-open No. 2005-230813

Non Patent Literature

Non Patent Literature 1: Toshihiko Hirao et. al., Appl. Microbiol. Biotechnol., 32, 269-273 (1989)

Summary

Technical Problem

An object of the present invention is to provide a hollow fiber membrane module for use in the production of a chemical substance by a continuous fermentation process capable of steam sterilization, in which microorganisms and the like are not accumulated inside hollow fiber membrane bundles and high productivity is stably maintained over a long period of time.

Solution to Problem

The present invention has the following configurations to achieve the object.

(1) A hollow fiber membrane module for use in production of a chemical substance, which is used in continuous fermentation including filtering a fermentation broth of a microorganism or a cultured cell through a hollow fiber membrane, collecting a chemical substance from a filtrate, retaining a concentrated solution in the fermentation broth or refluxing the concentrated solution, and adding a fermentation raw material to the fermentation broth, wherein a large number of hollow fiber membrane bundles are accommodated in a tubular case, at least one end part of each of the hollow fiber membrane bundles is fixed on the tubular case by a hollow fiber membrane bundling member with an end face of each of the hollow fiber membranes open, and the hollow fiber membrane bundling member is made of a synthetic resin having a hardness retention rate after contact with saturated steam at 121°C for 24 hours of 95% or more.

(2) The hollow fiber membrane module for use in the production of a chemical substance according to (1), wherein one end part of each of the hollow fiber membrane bundles is fixed on the tubular case by the hollow fiber membrane bundling member with the end face of each of the hollow fiber membrane open, the other end part of each of the hollow fiber membrane bundles is divided into a plurality of small bundles, and the end face of each of the hollow fiber membranes by the small bundle is plugged by a small bundle plugging member.

(3) The hollow fiber membrane module for use in the production of a chemical substance according to (1) or (2), wherein the hollow fiber membrane is obtained by bringing a hollow fiber membrane containing a fluororesin-based macromolecule into contact with saturated steam at 110°C or higher and 135°C or lower.

(4) The hollow fiber membrane module for use in the production of a chemical substance according to (1) or (2), wherein the hollow fiber membrane is obtained by bringing a hollow fiber membrane containing a fluororesin-based macromolecule into contact with saturated steam at 120°C or higher and 130°C or lower.

(5) The hollow fiber membrane module for use in the production of a chemical substance according to any of (1) to (4), wherein the hollow fiber membrane contains a polyvinylidene fluoride-based resin.

(6) The hollow fiber membrane module for use in the production of a chemical substance according to any of (1) to (5), wherein the hollow fiber membrane contains a hydrophilic macromolecule having at least one kind selected from a fatty acid vinyl ester, vinyl pyrrolidone, ethylene oxide, and propion oxide, or a cellulose ester.

(7) A process for production of a chemical substance using the hollow fiber membrane module for use in the production of a chemical substance according to any of (1) to (6).

Advantageous Effects of Invention

According to the present invention, the use of the above-described hollow fiber membrane module stably maintains high productivity over a long period of time and enables continuous fermentation capable of repeating sterilization treatment. Further, a chemical substance as a fermentation product can be stably produced at low cost broadly in a fermentation industry.

Brief Description of Drawings

FIG. 1 shows a schematic longitudinal cross-sectional view illustrating a hollow fiber membrane module used in the present invention in which both ends of each of hollow fiber membranes are fixed on a tubular case.

FIG. 2 shows a schematic longitudinal cross-sectional view illustrating a hollow fiber membrane module used in the present invention in which one end of each of hollow fiber membranes is fixed on a tubular case.

FIG. 3 shows a partially enlarged view of the hollow fiber membrane module of FIG. 2 illustrating a portion in which each of the hollow fiber membranes is divided into a plurality of small bundles and is plugged by a plugging member.

FIG. 4 is a schematic flow diagram illustrating a continuous fermentation apparatus of the present invention. Description of Embodiments

The hollow fiber membrane used in the present invention as a separation membrane will be described.

As a material for the hollow fiber membrane used in the present invention, an organic material and an inorganic material can be used. From the viewpoints of separation performance, water permeability, and fouling resistance, an organic macromolecular compound can be suitably used. Examples thereof may include a polyethylene-based resin, a polypropylene-based resin, a polyvinyl chloride-based resin, a polyvinylidene fluoride-based resin, a polysulfone-based resin, a polyether sulfone-based resin, a polyacrylonitrile-based resin, a cellulose-based resin, a cellulose triacetate-based resin, and the like. A mixture of resins containing these resins as a main component may be used. As used herein, the main component means that the component is contained in a content of 50% by weight or more, and preferably of 60% by weight or more. In the present invention, a polyvinyl chloride-based resin, a polyvinylidene fluoride-based resin, a polysulfone-based resin, a polyether sulfone-based resin, and a polyacrylonitrile-based resin are preferable, in which membrane formation using a solution is easy and which are excellent in physical durability and chemical resistance. A polyvinylidene fluoride-based resin or a resin containing the resin as a main component is most preferable since it is characterized by having chemical strength (particularly, chemical resistance) and physical strength.

As the polyvinylidene fluoride-based resin, a homopolymer of vinylidene fluoride is preferably used. As the polyvinylidene fluoride-based resin, a copolymer having vinylidene fluoride and a copolymerizable vinyl monomer may be used. Examples of the vinyl monomer copolymerizable with vinylidene fluoride may include tetrafluoroethylene, hexafluoropropylene, trichlorofluoroethylene, and the like.

Further preferable is a hollow fiber membrane containing a fluororesin-based macromolecule, which has both a three-dimensional network structure and a spherical structure, and contains a hydrophilic macromolecule having at least one kind selected from a fatty acid vinyl ester, vinyl pyrrolidone, ethylene oxide, and propylene oxide or a cellulose ester in the three-dimensional network structure in order to impart hydrophilicity to the membrane.

As used herein, the three-dimensional network structure is referred to as a structure in which a solid content spreads in the form of a three-dimensional net. The three-dimensional network structure has a micropore and a void, which are partitioned by the solid content forming a net.

Further, the spherical structure is referred to as a structure in which many spherical or nearly spherical solid contents are connected directly or through a string-shaped solid content.

Moreover, the hollow fiber membrane is not particularly limited as long as it has both a spherical structure layer and a three-dimensional network structure layer. It is preferable that the hollow fiber membrane have lamination of the spherical structure layer and the three-dimensional network structure layer. In general, when a large number of layers are laminated, the respective layers penetrate into the other at the interface between the layers to become dense, and thus permeability deteriorates. When the respective layers do not penetrate into the other, the permeability does not deteriorate, but peel strength of the interface decreases. Therefore, in view of the peel strength and permeability at the interface between the layers, it is preferable that the laminating number of the spherical structure layer and the three-dimensional network structure layer be small. It is particularly preferable that the number be two in total, one spherical structure layer and one three-dimensional network structure layer. Furthermore, the hollow fiber membrane may contain for example, a support layer made of a porous substrate in addition to the spherical structure layer and the three-dimensional network structure layer. The porous substrate includes, but is not particularly limited to, an organic material, an inorganic material, and the like. From the viewpoints of easiness of weight saving, an organic fiber is preferable. Woven fabrics or nonwoven fabrics made of an organic fiber such as a cellulose-based fiber, a cellulose acetate-based fiber, a polyester-based fiber, a polypropylene-based fiber, and a polyethylene-based fiber are more preferable.

The top and bottom or inside and outside positions of a three-dimensional network structure layer and a spherical structure layer can vary depending on a filtration process. Since the three-dimensional network structure layer mainly has a separation function and the spherical structure layer mainly has a physical strength, it is preferable that the three-dimensional network structure layer be disposed on a separation object side. In particular, in order to suppress reduction of permeability caused by trapped contaminants, it is preferable that a three-dimensional network structure layer having a separation function be disposed on an outermost surface layer on a separation object side.

With respect to each thickness of a three-dimensional network structure layer and a spherical structure layer, each performance of fouling resistance suitable for filtration of a broth, separation property, water permeability, physical strength, and chemical strength (chemical resistance) should be considered. When a three-dimensional network structure layer is thin, the fouling resistance, separation property, and physical strength are low, and when it is thick, the water permeability is low. Therefore, in view of balance of the respective performances, the thickness of a three-dimensional network structure layer is preferably 5 $\mu$m or more and 50 $\mu$m or less, and more preferably 10 $\mu$m or more and 40 $\mu$m or less. The thickness of a spherical structure layer is preferably 100 $\mu$m or more and 500 $\mu$m or less, and more preferably 200 $\mu$m or more and 300 $\mu$m or less. Further, the ratio of thickness of a three-dimensional network structure layer to a spherical structure layer is important for the respective performances. When the ratio of a three-dimensional network structure layer to a spherical structure layer is large, the physical strength decreases. Accordingly, the ratio of the average thickness of a three-dimensional network structure layer to the average thickness of a spherical structure layer is preferably 0.03 or more and 0.25 or less, and more preferably 0.05 or more and 0.15 or less.

An interface between a spherical structure and a three-dimensional network structure has a structure in which the both structures are in the other structures each other. A spherical structure layer is referred to as a layer within a range where a spherical structure is observed when a cross-section of a macromolecular separation membrane is photographed under a scanning electron microscope at a magnification of 3,000 times. A three-dimensional network structure layer is referred to as a layer within a range where a spherical structure is not observed when a cross-section of a macromolecular separation membrane is photographed under a scanning electron microscope at a magnification of 3,000 times.

When the average diameter of a spherical structure is too large, the porosity increases, and therefore the water permeation property increases and the physical strength deteriorates. On the other hand, when the average diameter is too small, the porosity is low, and therefore the physical strength increases and the water permeation property deteriorates. Accordingly, the average diameter of the spherical structure is preferably 0.1 $\mu$m or more and 5 $\mu$m or less, and more preferably 0.5 $\mu$m or more and 4 $\mu$m or less. The average diameter of the spherical structure is determined by photographing a cross-section of a macromolecular separation membrane under a scanning electron microscope at a magnification of 10,000 times, measuring the diameters of 10 or more, and preferably 20 or more any spherical structures, and number-averaging the diameters. The average diameters of spherical structures is calculated with an image processing apparatus and the like, and it is preferably utilized as the average diameter of an equivalent circular diameter.

When a three-dimensional network structure is disposed on an outermost surface layer on a separation object side, the surface of the outermost surface layer is observed from directly above this layer, and micropores are observed. The average pore diameter of surface of a three-dimensional network structure is preferably 0.1 nm or more and 1 $\mu$m or less, and more preferably 5 nm or more and 0.5 $\mu$m or less in order to achieve high inhibition performance and high water permeability. Further, microorganisms or cultured cells sometimes produce substances other than the target chemical substance, for example, aggregating matters such as proteins and polysaccharides, and some of the microorganisms or cultured cells in the broth are extinct and fracturing matters of cells are sometimes produced. In order to prevent blocking of these matters on a porous membrane, the average pore diameter of surface of a three-dimensional network structure preferably falls within a range of 5 nm or more and 0.5 $\mu$m or less, and more preferably a range of 0.02 $\mu$m or more and 0.2 $\mu$m or less.

When the average pore diameter of the surface is within this range, the micropore is unlikely to be blocked with dirt matters in water, and water permeability is unlikely to deteriorate. Therefore, a macromolecular separation membrane can be continuously used over a long period of time. When the micropore is blocked, dirty can be removed by so-called backwashing or air-washing. Examples of the dirt matters may include a microorganism, a carcass thereof, an unfermented remaining culture medium, a by-product of fermentation, proteins generated by fermentation or cultivation, and the like. The backwashing is an operation in which permeated water and the like pass in a backward direction of ordinal filtration. The air-washing is an operation in which air is send to swing a hollow fiber membrane and thus dirt matters accumulated on a membrane surface are removed.

The average pore diameter of a three-dimensional network structure is determined by photographing the surface of the three-dimensional network structure under a scanning electron microscope at a magnification of 60,000 times, measuring the diameters of 10 or more, and preferably 20 or more of arbitrarily selected micropores, and number-averaging the diameters. When the micropore is not circle, a circle (equivalent circle) having an area equivalent to the area of the micropore is determined with an image processing apparatus and the like, and the average pore diameter is determined through a method using the diameter of the equivalent circle as the diameter of the micropore.

It is preferable that in a fluororesin-based macromolecular separation membrane having a three-dimensional network structure and a spherical structure, the three-dimensional network structure is characterized by containing a hydrophilic macromolecule having at least one kind selected from a fatty acid vinyl ester, vinyl pyrrolidone, ethylene oxide, and propylene oxide, or a cellulose ester. A fluororesin-based macromolecule is referred to as a resin containing a vinylidene fluoride homopolymer and/or a vinylidene fluoride copolymer. The macromolecule may contain a plurality of kinds of vinylidene fluoride copolymers. Examples of the vinylidene fluoride copolymers may include a copolymer of at least one kind selected from vinyl fluoride, ethylene tetrafluoride, propylene hexafluoride, and ethylene chloride trifluoride with vinylidene fluoride.

Further, the weight average molecular weight of a fluororesin-based macromolecule may be appropriately selected depending on the strength and water permeability of a desired macromolecular separation membrane. When the weight average molecular weight is large, the water permeability deteriorates, and when the weight average molecular weight is small, the strength deteriorates. Therefore, the weight average molecular weight is preferably 50,000 or more and 1,000,000 or less. In particular, suppose a case where a fermentation liquid is filtered by an operation, and dirt matters adhered to a separation membrane need to be removed by chemical cleaning so that the fermentation liquid is filtered again. In this case, the weight average molecular weight is preferably 100,000 or more and 700,000 or less. When the chemical cleaning is repeated a plurality of times, it is more preferably 150,000 or more and 600,000 or less.

The hydrophilic macromolecule having at least one kind selected from a fatty acid vinyl ester, vinyl pyrrolidone, ethylene oxide, and propylene oxide or the cellulose ester is not particularly limited as long as it is a compound having at least one kind selected from a fatty acid vinyl ester, vinyl pyrrolidone, ethylene oxide, and propylene oxide or a cellulose ester in a main chain and/or a side chain as a molecular unit (herein, in the case of a fatty acid vinyl ester, vinyl pyrrolidone, ethylene oxide, or propylene oxide, it means that the compound having a molecular unit derived using it as a monomer). Further, molecular units other than these compounds may be present. Examples of a monomer constituting the molecular unit other than a fatty acid vinyl ester, vinyl pyrrolidone, ethylene oxide, propylene oxide, and a cellulose ester may include alkene such as ethylene and propylene, alkyne such as acetylene, vinyl halide, vinylidene halide, methyl methacrylate, methyl acrylate, and the like. Since the hydrophilic macromolecule is used together with a fluororesin-based macromolecule to form a three-dimensional network structure, it is preferable that a hydrophilic macromolecule be mixed with a fluororesin-based macromolecule under an appropriate condition. Further, when a hydrophilic macromolecule and a fluororesin-based macromolecule are mixed and dissolved in a good solvent for the fluororesin-based macromolecule, handling becomes easy, and therefore it is particularly preferable.

When the content of a fatty acid vinyl ester, vinyl pyrrolidone, ethylene oxide, propylene oxide, or a cellulose ester which is a hydrophilic macromolecule increases, the hydrophilicity of the obtained macromolecular separation membrane increases, and the permeability and the fouling resistance are improved. Therefore, it is preferable that the content be higher within a range in which the miscibility with a fluororesin-based macromolecule is not lost. The content of a fatty acid vinyl ester, vinyl pyrrolidone, ethylene oxide, propylene oxide, or a cellulose ester in a hydrophilic macromolecule depends on a ratio mixed with a fluororesin-based macromolecule, and a performance of a desired macromolecular separation membrane is preferably 50% by mole or more, and more preferably 60% by mole or more.

It is particularly preferable that in a fluororesin-based macromolecular separation membrane having both a three-dimensional network structure and a spherical structure, the three-dimensional network structure mainly contain a hydrophilic macromolecule including a cellulose ester and/or a fatty acid vinyl ester. This is because in the case of a constitution mainly including a cellulose ester and/or a fatty acid vinyl ester, the degree of hydrolysis of ester can be extensively adjusted within a range in which the miscibility with a fluororesin-based macromolecule is not lost, and the obtained macromolecular separation membrane is likely to be provided with hydrophilicity. The hydrophilic macromolecule mainly including a cellulose ester and/or a fatty acid vinyl ester is referred to as a hydrophilic macromolecule in which the content of the cellulose ester or the fatty acid vinyl ester is 70% by mole or more, or the sum of the content of the cellulose ester and the content of the fatty acid vinyl ester is 70% by mole or more, and more preferably 80% by mole or more.

In particular, the cellulose ester is preferably used since it has three ester groups in a repeating unit, the degree of hydrolysis thereof is adjusted to easily achieve both the miscibility with a fluororesin-based macromolecule and hydrophilicity of the macromolecular separation membrane. Examples of the cellulose ester may include cellulose acetate, cellulose acetate propionate, and cellulose acetate butyrate.

Examples of the fatty acid vinyl ester may include a homopolymer of a fatty acid vinyl ester, a copolymer of a fatty acid vinyl ester with another monomer, and a polymer obtained by graft polymerization of a fatty acid vinyl ester with another polymer. As the homopolymer of a fatty acid vinyl ester, polyvinyl acetate is preferably used because of inexpensiveness and easy handling. As the copolymer of a fatty acid vinyl ester with another monomer, an ethylene-vinyl acetate copolymer is preferably used because of inexpensiveness and easy handling.

Further, the three-dimensional network structure and the spherical structure may contain other components within a range in which cultivation is not inhibited, such as an organic substance, an inorganic substance, a macromolecule, and the like.

An outline of a process for production of a fluororesin-based hollow fiber membrane having a three-dimensional network structure and a spherical structure will be described. First, in a process for production of a fluororesin-based hollow fiber membrane having a spherical structure, a fluororesin-based macromolecule in a concentration as relatively high as about 20% by weight to about 60% by weight or lower is dissolved in a poor solvent or a good solvent for the macromolecule at relatively high temperature to prepare a macromolecular solution. After the preparation of the macromolecular solution, while the macromolecular solution is discharged from the outside of a double pipe sleeve and at the same time a hollow section-forming fluid is discharged from a pipe inside the double pipe sleeve, the macromolecular solution is solidified in a cooling bath to form a hollow fiber membrane. At this time, the hollow section-forming fluid can be usually used in a gas or liquid form. It is preferable that as the hollow section-forming fluid, a liquid containing a poor solvent or a good solvent in the same concentration as in a cooling liquid of 60% by weight or more and 100% by weight or less be used. The hollow section-forming fluid may be cooled and then supplied. Further, when only the cooling power of a cooling bath is sufficient to solidify a hollow fiber membrane, the hollow section-forming fluid may be supplied without cooling.

As used herein, the poor solvent is a solvent which is not capable of dissolving 5% by weight or more of macromolecule at a low temperature of 60°C or lower, but is capable of dissolving 5% by weight or more of macromolecule at a high temperature region in which the temperature is 60°C or higher and a melting point of the macromolecule or lower (for example, when the macromolecule is composed of a vinylidene fluoride homopolymer alone, it is about 178°C). In contrast to the poor solvent, a solvent which is capable of dissolving 5% by weight or more of macromolecule at a low temperature region of 60°C or lower is defined as a good solvent. A solvent which is not capable of dissolving and swelling the macromolecule until the melting point of the macromolecule or the boiling point of the solvent is defined as a non-solvent.

On a fluororesin-based macromolecular separation membrane having a spherical structure as obtained above, a three-dimensional network structure containing a hydrophilic macromolecule having at least one kind selected from a fatty acid vinyl ester, vinyl pyrrolidone, ethylene oxide, and propylene oxide, or a cellulose ester is laminated. A method for lamination is not particularly limited, but the following method can be preferably used. The method is a method in which a fluororesin-based macromolecular solution containing the hydrophilic macromolecule is applied to the fluororesin-based macromolecular separation membrane having a spherical structure, and immersed in a coagulation bath to laminate a layer having a three-dimensional network structure.

The fluororesin-based macromolecule solution containing a hydrophilic macromolecular having at least one kind selected from a fatty acid vinyl ester, vinyl pyrrolidone, ethylene oxide, and propylene oxide, or a cellulose ester for formation of a three-dimensional network structure is composed of the hydrophilic macromolecule, a fluororesin-based macromolecule, and a solvent. As the solvent, the good solvent for a fluororesin-based macromolecule is preferably used. The macromolecular concentration of a fluororesin-based macromolecular solution containing the hydrophilic macromolecule usually falls within a range of preferably 5% by weight or more and 30% by weight or less, and more preferably 10% by weight or more and 25% by weight or less. When the concentration is less than 5% by weight, the physical strength of the three-dimensional network structure layer deteriorates. When it exceeds 30% by weight, the permeability deteriorates. Further, the dissolving temperature of the fluororesin-based macromolecular solution containing the hydrophilic macromolecule varies depending on the kind and concentration of the fluororesin-based macromolecule and the hydrophilic macromolecule, the kind of the solvent, and the kind and concentration of an additive. In order to prepare a good reproducible and stable solution, it is preferable that the solution be heated for several hours while stirred at a temperature equal to or lower than the boiling point of the solvent to become transparent. In addition, the temperature during the application of the solution is important. In order to stably produce a macromolecular separation membrane, it is preferable that the temperature be controlled so as not to lose the stability of the solution and a non-solvent be prevented from intruding from the outside of a system. When the application temperature of the solution is too high, the fluororesin-based macromolecular separation membrane including a spherical structure is dissolved, a dense layer is likely to be formed on the interface between a three-dimensional network structure layer and a spherical structure layer, and therefore water permeability deteriorates. On the contrary, when the application temperature of the solution is too low, the solution is partially converted into gel during the application to form a separation membrane containing many disadvantages. Therefore, the separation performance deteriorates. For this reason, the application temperature needs to be intensively studied and determined according to the composition of the solution and the performance of a desired separation membrane.

In the case of a hollow fiber membrane, as a process for applying a fluororesin-based macromolecular solution containing a hydrophilic macromolecule having at least one kind selected from a fatty acid vinyl ester, vinyl pyrrolidone, ethylene oxide, and propylene oxide, or a cellulose ester to a fluororesin-based macromolecular separation membrane including a spherical structure, a process for immersing the hollow fiber membrane into the macromolecular solution or adding dropwise the macromolecular solution to the hollow fiber membrane is preferably used, and as a process for applying the macromolecular solution to the inner surface of the hollow fiber membrane, a process for injecting the macromolecular solution into the hollow fiber membrane, and the like are preferably used. Further, as a process for controlling the application amount of the macromolecular solution, a process in which the macromolecular separation membrane is immersed in the macromolecular solution or the macromolecular solution is applied to the membrane and then the macromolecular solution is partially scraped off or blown off with an air knife is preferably used, in addition to control of the application amount itself of the macromolecular solution.

Further, as used herein, the coagulation bath preferably contains a resin as a non-solvent. As the non-solvent, the above-described substances can be preferably used. The applied resin solution is brought into contact with the non-solvent to generate a non-solvent-induced phase separation, and a three-dimensional network structure layer is formed.

With respect to this hollow fiber membrane, a treatment of bringing into contact with saturated steam is preferably performed before or after the assembly of a hollow fiber membrane module. When contraction of the hollow fiber membrane by the treatment of bringing into contact with saturated steam is large, there is a concern that the membrane area per module decreases and adhesion between a hollow fiber membrane bundling member and the hollow fiber membrane lowers due to the contraction of the hollow fiber membrane in the hollow fiber membrane bundling member. Therefore, it is preferable that the hollow fiber membrane be brought into contact with saturated steam before the assembly of a hollow fiber membrane module. For example, when the hollow fiber membrane is brought into contact with saturated steam after the assembly of a hollow fiber membrane module and the hollow fiber membrane is contracted in a lengthwise direction by 10%, the membrane area in the hollow fiber membrane module decreases by 10%. Therefore, in order to have the same membrane area by the process for bringing into contact with saturated steam after the assembly of a hollow fiber membrane module, the length of the hollow fiber membrane needs to be previously retained longer. During bringing the hollow fiber membrane into contact with saturated steam, the hollow fiber membrane may have fluidity in a glass, rubber, or liquid state at a contact temperature depending on the material and composition of the hollow fiber membrane, and the surface structure of the hollow fiber membrane may vary. For this reason, the fine micropore diameter of the surface of the hollow fiber membrane increases, and the filterability of the fermentation liquid may be improved.

The contact temperature with saturated steam is preferably 110°C or higher and 135°C or lower, and more preferably 120°C or higher and 130°C or lower. When the contact temperature with saturated steam exceeds 140°C, the temperature may be close to the melting point of a fluororesin, and there is a concern that the surface roughness increases or the micropore is damaged.

Further, since the hollow fiber membrane takes time to change by the contact with saturated steam, the contact time with saturated steam needs to be longer than a certain time. The contact time varies depending on the quality of material and composition of a hollow fiber membrane, and the like. However, from the viewpoints of save energy and productivity, it is preferable that the contact time be minimum requirement. For example, in the case of polyvinylidene fluoride, the lengths of a hollow fiber membrane in a lengthwise direction and a radial direction are each approximately the same as those after bringing into contact with saturated steam at 121°C for 1 hour or more.

The saturated steam used herein is in a state in which a further amount of steam is not contained at a predetermined temperature and steam is saturated. With respect to the saturated steam used in the present invention, for example, an autoclave in which an object is enclosed with excess water in a liquid state kept is heated in a closed state with an electric heater or the like, and the steam becomes saturated. The object is then brought into contact with the saturated steam at a determined temperature for a determined time. At this time, the inside of the autoclave in a closed state is in a pressurized state, and the temperature and the pressure are determined from the relation of saturated steam pressure. For example, they are 121°C and about 0.21 MPa. Further, there is a process for bringing an object into contact with saturated steam using a high-pressure steam generated by a boiler or the like by enclosing the object into a heat-resistant and pressure-resistant container and supplying the high-pressure steam.

The contact with saturated steam can be performed in a batchwise manner or a continuous manner. In the continuous manner, the contact of objects to be continuously supplied can be performed in a space under a saturated steam atmosphere. In the space under the saturated steam atmosphere, a saturated state of steam may be produced by heating with excess water in a liquid state kept just like an autoclave when desired temperature and pressure can be maintained by appropriate sealing. Alternatively, the contact with saturated steam may be performed by supplying high-pressure steam and continuously removing drain water generated by heat exchange with a steam trap and the like.

In the hollow fiber membrane used in the present invention, difficulty of clogging against a fermentation broth, that is, fouling resistance is one of important performances. For this reason, the balance of the average micropore diameter and pure water permeability of the hollow fiber membrane is important. It is preferable that the average micropore diameter be small enough not to include dirt matters of a membrane inside the micropore. On the other hand, when the micropore is small, the water permeability deteriorates, and therefore a transmembrane pressure difference during a filtration operation increases and a stable operation cannot be performed. Thus, it is preferable that the water permeability be rather higher. As the indicator of the water permeability, a pure water permeation coefficient of a hollow fiber membrane before use can be used. In the present invention, a water permeation volume at a head height of 1 m is measured using purified water at 25°C obtained by reverse osmosis membrane filtration, and the pure water permeation coefficient of a hollow fiber membrane is calculated. The pure water permeation coefficient at this time is $5.6 \times 10^{-10}$ $m^3/m^2/s/Pa$ or more and $1.6 \times 10^{-8}$ $m^3/m^2/s/Pa$ or less, preferably $1.1 \times 10^{-9}$ $m^3/m^2/s/Pa$ or more and $1.3 \times 10^{-8}$ $m^3/m^2/s/Pa$ or less, and more preferably $1.7 \times 10^{-9}$ $m^3/m^2/s/Pa$ or more and $1.1 \times 10^{-8}$ $m^3/m^2/s/Pa$ or less.

The average micropore diameter can be appropriately determined according to purpose and situation to be used when the water permeability falls within the above-described range. The average micropore diameter is preferably rather smaller, and may be usually 0.01 $\mu$m or more and 1 $\mu$m or less. When the average micropore diameter of a hollow fiber membrane is less than 0.01 $\mu$m, a membrane fouling component such as a component including saccharides and proteins and an aggregated body thereof blocks the micropores, and a stable operation cannot be performed. In view of balance with the water permeability, the average micropore diameter is preferably 0.02 $\mu$m or more, and more preferably 0.03 $\mu$m or more. When it exceeds 1 $\mu$m, the dirt component is insufficiently peeled from the micropores by shear power due to smoothness of and flow on a membrane surface and physical cleaning such as backwashing and air scrubbing. Therefore, a stable operation cannot be performed. Further, when the average micropore diameter of a hollow fiber membrane is closed to the size of a microorganism or a cultured cell, the microorganism or the cultured cell may directly block the micropores. Further, when some microorganisms or cultured cells in a fermentation broth are sometimes killed to produce fracturing matters of the cells, the average micropore diameter is preferably 0.4 $\mu$m or less to prevent blocking on a hollow fiber membrane. When the average micropore diameter is 0.2 $\mu$m or less, an operation can be more suitably performed.

The average micropore diameter can be calculated by measuring the diameters of a plurality of micropores observed under a scanning electron microscope at a magnification of 10,000 times or more and averaging the diameters. It is preferable that the average micropore diameter be calculated by randomly selecting 10 or more, and preferably 20 or more micropores, measuring the diameters of these micropores, and number-averaging the diameters. When the micropore is not circle, it is preferable that a circle having an area equivalent to the area of the micropore, or an equivalent circle, be determined with an image processing apparatus and the like, and the average micropore diameter be determined through a method using the diameter of the equivalent circle as the diameter of the micropore.

The outer diameter of the hollow fiber membrane used in the present invention is preferably 0.6 mm or more and 2.0 mm or less, and more preferably 0.8 mm or more and 1.8 mm or less. When a hollow fiber membrane having an inner diameter less than 0.6 mm is used, the effective membrane area increases, and therefore more chemical substances can be filtered. However, when the hollow fiber membrane is accommodated in a module or a fermentation broth is circulated in the module, the hollow fiber membrane is broken or ruptured by external forces to mix the fermentation broth in a filtrate. In this respect, it is not preferable. Further, it is not preferable that a fine hollow fiber membrane be used since microorganisms enter inside a hollow fiber membrane bundle and a phenomenon which is difficult to discharge them occurs. When a hollow fiber membrane having an inner diameter more than 2.0 mm is used, a risk for breaking or rupturing the hollow fiber membrane is low. However, when the hollow fiber membrane is added to a module having the same volume, the effective membrane area decreases, and the filtration amount per unit volume decreases. Therefore, it is not preferable. In addition, swinging property of the hollow fiber membrane deteriorates and discharging property of microorganisms and dirt components from the inside of a separation membrane module deteriorates. Therefore, it is not preferable.

The configuration of the hollow fiber membrane used in the present invention may be an external pressure type hollow fiber membrane or an inner pressure type hollow fiber membrane. When a microorganism used in fermentation has low dispersibility and flocks are formed, there is a concern that use of the inner pressure type hollow fiber membrane causes clogging with fermentation liquid flowing on a primary side of a separation membrane. Thus, it is preferable that the external pressure type hollow fiber membrane be used.

The rupture strength of the hollow fiber membrane used in the present invention is preferably 6 MPa or larger, and more preferably 7 MPa or larger. When the rupture strength is less than 6 MPa, the hollow fiber membrane may not endure swinging during physical cleaning such as flushing and air scrubbing, and the rupture of the hollow fiber membrane is concerned. Therefore, it is not preferable. Further, the rupture elongation percentage of the hollow fiber membrane used in the present invention is preferably 20% or more. In the case of a rupture elongation percentage less than 20%, when fibers are forcedly swung by flushing and air scrubbing like the rupture strength, the probability of rupture of a membrane is high. Therefore, this is not preferable.

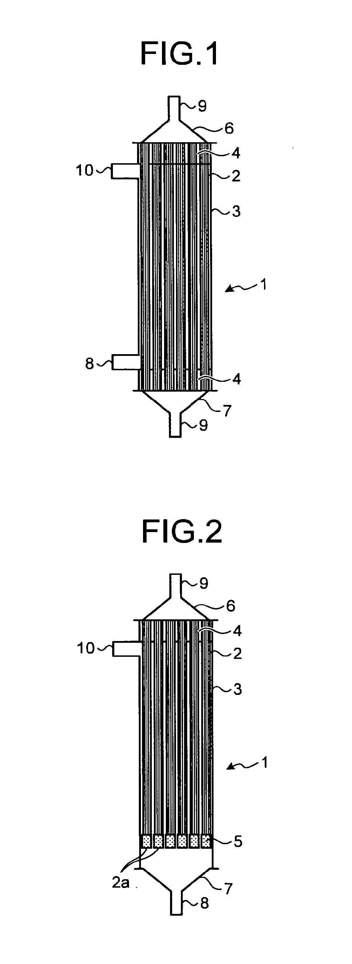

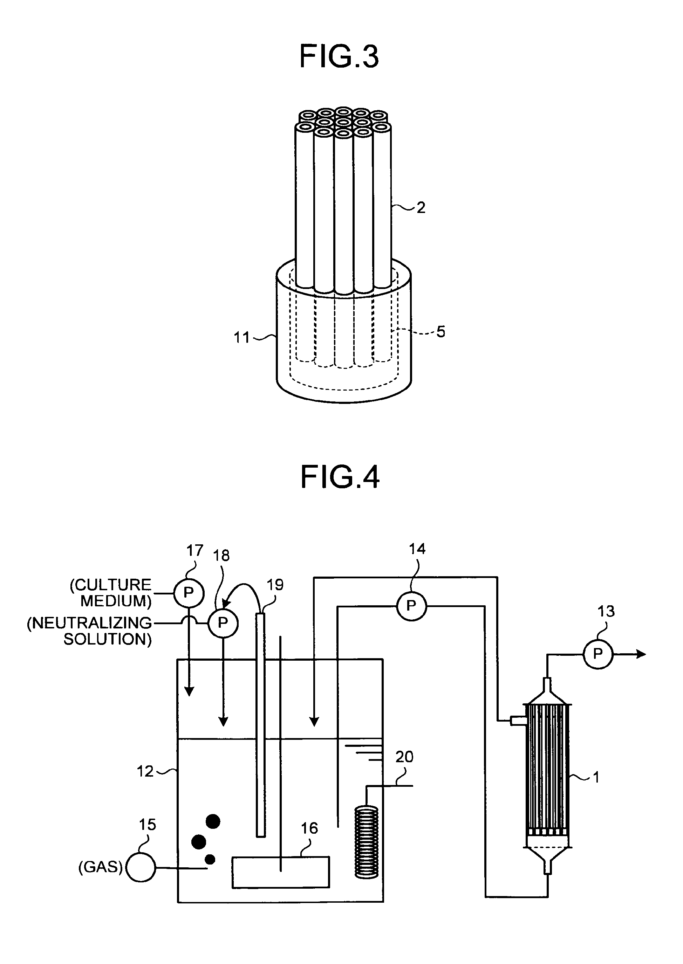

The configuration of the hollow fiber membrane module in the present invention will be described with reference to the drawings. FIG. 1 shows a schematic longitudinal cross-sectional view of a hollow fiber membrane module in which at least one end part of each of hollow fiber membrane bundles is fixed on a tubular case by a hollow fiber membrane bundling member with the end face of each of hollow fiber membranes open. FIG. 2 shows a schematic longitudinal cross-sectional view of a hollow fiber membrane module in which one end part of each of hollow fiber membrane bundles is fixed on a tubular case by a hollow fiber membrane bundling member with the end face of each of hollow fiber membranes open, another end part of each of the hollow fiber membrane bundles is divided into a plurality of small bundles, and the end face of each of the hollow fiber membranes by the small bundle is plugged by a small bundle plugging member. FIG. 3 shows a schematic perspective view illustrating a portion in which each of the hollow fiber membranes is divided into a plurality of small bundles and plugged by the clogging member.

As shown in FIG. 1, a hollow fiber membrane module 1 may be a configuration of a module in which a large number of hollow fiber membranes 2 are accommodated in a tubular case 3 of which both ends are open, both end parts of each of hollow fiber membrane bundles are fixed on the tubular case 3 by a hollow fiber membrane bundling member 4 with at least one of end face of each of the hollow fiber membranes 2 open. Further, as shown in FIG. 2, a large number of hollow fiber membranes 2 are accommodated in a tubular case 3 of which both ends are open, an upper end part of each of the hollow fiber membranes 2 is fluid-tightly fixed on the top end of the tubular case 3 by a hollow fiber membrane bundling member 4 with the end face of each of the hollow fiber membranes 2 open, a lower end part of each of the hollow fiber membranes 2 is divided into 3 to about 300 small bundles 2a and the end face may be gathered by the small bundle 2a and plugged by a small bundle plugging member 5. Moreover, each of the hollow fiber membranes 2 can freely move in the lower end part by the small bundle 2a. When each of the hollow fiber membranes 2 freely moves by the small bundle 2a, a bent part of the bundle in which the bundle is bent in a U-shaped form may be gathered by the small bundle plugging member 5. For reinforcement, each small bundle 2a may contain fiber-like or rod-like materials having high strength and low ductility such as a steel wire and an aramid fiber cord.

An upper cap 6 having a filtered liquid outlet 9 and a lower cap 7 having an inlet 8 of fermentation broth and air are fluid-tightly connected with the upper part and the lower part of the tubular case 3, respectively. A fermentation broth not passing through the hollow fiber membrane 2 is discharged from a fermentation broth outlet 10 outside of the hollow fiber membrane module 1.

As the material for the tubular case 3, the upper cap 6, and the lower cap 7, a material having heat resistance against steam sterilization is required and for example, heat-resistant resins such as polysulfone, polycarbonate, and polyphenylene sulfide are used alone or in combination. In addition to the resin, aluminum, stainless steel, and the like are preferable. Further, a composite of resin and metal, and a composite material such as a glass fiber-reinforced resin and a carbon fiber-reinforced resin may be used.

In the present invention, the hollow fiber membrane bundling member 4 by which a bundle of the hollow fiber membrane 2 is fixed on the tubular case 3 and the small bundle plugging member 5 by which the hollow fiber membrane 2 is gathered by the small bundle 2a and closed require heat resistance against steam sterilization. The inventors have intensively studied, and as a result, found that the retention of hardness of a hardened material obtained after 24 hours under a saturated steam condition of 121°C and 2 atmospheric pressure needs to have 95% or more.

When the hollow fiber membranes 2 are gathered into the small bundle 2a and fixed on the tubular case 3 by the hollow fiber membrane bundling member 4 and small bundle plugging member 5, they exert a function of the hollow fiber membrane module 1. However, when the hardness is too low, the hardened material is too soft. Therefore, during an operation such as filtration or counter-pressure cleaning or during steam sterilization, when a differential pressure exists between the primary and secondary sides of a separation membrane by supplying high pressure-saturated steam from the primary side of a separation membrane, there is a concern that the hollow fiber membrane bundling member 4 is largely deformed, peeling occurs on an adhesion interface, and the hollow fiber membrane 2 is ruptured to develop leaks. Further, in a case where the hardness is too high, when the hollow fiber membrane swings during an operation such as filtration or counter-pressure cleaning, the hollow fiber membrane 2 has a strong tendency to be damaged or ruptured on an adhesion interface between the hollow fiber membrane bundling member 4 or the small bundle plugging member 5 and the hollow fiber membrane 2. As described above, the hardness is an important factor for functions of the hollow fiber membrane bundling member 4 and the small bundle plugging member 5. If the steam sterilization is repeated, the hollow fiber membrane bundling member 4 and the small bundle plugging member 5 do not react and degrade, and need to be stable. The retention of hardness of a hardened material obtained after 24 hours under a saturated steam condition of 121°C and 2 atmospheric pressure needs to have 95% or more.

In order to prevent dropped pick or damage or rupture of fibers on the interfaces between the hollow fiber membrane bundling member 4 and the small bundle plugging member 5 and the hollow fiber membrane 2, a synthetic resin having a type D durometer hardness after hardening of about 50 or more and about 80 or less according to JIS-K6253(2004) may be used as the hollow fiber membrane bundling member 4 and the small bundle plugging member 5. Further, as the hollow fiber membrane bundling member 4 and the small bundle plugging member 5, the use of a synthetic resin such as an epoxy resin or a polyurethane resin, which is a resin for general purpose application and inexpensive, and does not affect water quality, is preferable.

As a method for confirming that the hardened material obtained after 24 hours under a saturated steam condition of 121°C and 2 atmospheric pressure has a retention of hardness of 95% or more, for example, the following method can be used.

30 g of polyurethane resin (available from SANYU REC CO., LTD., two liquids, SA-7068A and SA-7068B, are mixed so that a weight ratio is 64:100) is sufficiently stirred and mixed, and is then hardened at 80°C for 4 hours to be formed into a plate having a thickness of 6 mm. The resin stands at room temperature and is naturally cooled to 25°C, and the D hardness is measured with a type D durometer according to JIS-K6253. The type D durometer hardness according to JIS-K6253(2004) is measured using a measuring device according to JIS-K6253(2004) by pushing a probe into the surface of the resin.

The resin of which the hardness is measured is heat-treated under a saturated steam condition of 121°C and 2 atmospheric pressure for 24 hours and naturally cooled to 25°C, moisture on the surface is sufficiently removed, and the hardness is measured by the above-described method. The heat treatment can be performed using an autoclave or a pressure cooker. The heat treatment may be 24 continuous hours or an accumulated total of 24 hours. However, repetition steam sterilization is assumed to be performed during the actual use, and therefore it is preferable that the repetition steam sterilization be performed for the accumulated total of 24 hours.

The small bundle plugging member 5 can have various shapes depending on a packing density of the hollow fiber membrane 2 in the tubular case 3, a size of space between the small bundles 2a, and the like. For example, the shape can be a column, spherical, or streamline shape. In addition, the shape may be a conical, pyramid, or platelike shape. Further, in the case of the column shape, it is preferable that a cross-sectional shape is circular since molding is easy and damage is less likely to be suffered. In addition, the cross-sectional shape may be a polygon such as a triangle, a rectangle, a pentagon, and a hexagon, an ellipse, a star shape, and the like.

When a resin is used as the small bundle plugging member 5, the end part of the hollow fiber membrane 2 is disposed in a container 11 as shown in FIG. 3, and the resin is poured in the container 11 and solidified, whereby the end face of the hollow fiber membrane 2 is plugged. Thus, a more efficient closing operation is possible, and further a weight drop effect due to the container can be obtained since the container is used as it is as a module. Herein, it is preferable that the material for the container 11 be a heat resistant resin such as polysulfone, or stainless steel (SUS).

In order to prevent decrease of filling rate of the hollow fiber membrane 2 into the tubular case 3 by the small bundle plugging member 5, the position of adjacent small bundle plugging members 5 may be shifted in an axial direction of the hollow fiber membrane module 1 (vertically).

Further, each small bundle plugging member 5 in which the end face of the hollow fiber membrane 2 is plugged by the small bundle 2a may be partially connected with each of adjacent small bundle plugging members 5. For example, this is a structure in which each of the small bundle plugging members 5 is connected by a rod-like body or a cord-like body. Since such a structure is one in which the small bundle plugging members 5 are linked to each other, only a small bundle plugging member 5 at a specific position does not swing, and vibration and swinging forces can be transmitted to another small bundle plugging member 5. At the same time, the position of each of the small bundles 2a can be moderately controlled, and the dispersibility of raw water and air can be improved. Therefore, effects of preventing occurrence of dirt spots and effects of preventing the small bundles 2a from entangling each other can be improved.

Herein, the filling rate of the hollow fiber membrane in the hollow fiber membrane module of the present invention will be calculated by a following expression 1. The filling rate of the hollow fiber membrane in the hollow fiber membrane module can be appropriately determined depending on the use purpose and state, and in general, is preferably 30% or more and 50% or less, and more preferably 35% or more and 48% or less. The present invention requires circulation of a fermentation broth between a fermenter and a hollow fiber membrane module to retain or reflux microorganisms or cultured cells in the fermentation broth by continuous fermentation. However, when the linear speed of the fermentation broth on a membrane surface inside the hollow fiber membrane module is low, shear force due to flow on the membrane surface decreases, and therefore a micropore or the inside of a small bundle clog with dirt matters of the membrane and a stable operation is difficult. Since a membrane area per the hollow fiber membrane module decreases, the production efficiency of a chemical substance also reduces. When the filling rate is less than 30%, the cross-section area except for the hollow fiber membrane in the hollow fiber membrane module increases. Therefore, in order to increase a membrane surface linear speed, a circulation flow rate needs to increase. When the circulation flow rate increases, a circulation pump is large, and therefore equipment cost increases and electric power for the operation increases. Further, when the circulation flow rate increases, pipes need to become thick. Therefore, there is a problem of high cost for the pipes and valves such as an automatic valve. When the filling rate increases, a flow pass cross-sectional area of a fermentation broth decreases. Therefore, this is advantageous since a small circulation flow rate causes a high membrane surface linear speed. However, when the filling rate exceeds 50%, the swinging property of a small bundle deteriorates, and the proportion of volume of a hollow fiber membrane in a hollow fiber membrane module increases. Therefore, the discharging property of microorganisms deteriorates, and clogging of the membrane easily occurs. Further, when the filling rate exceeds 50%, it is difficult to insert the hollow fiber membrane into the container of the hollow fiber membrane module, and the filling rate of the hollow fiber membrane in a small bundle plugging member part increases. Therefore, the small bundle plugging member is difficult to penetrate between the hollow fiber membranes, and there is a problem of difficulty of production of small bundles. Moreover, when the hollow fiber membrane has a lopsided position in the hollow fiber membrane bundling member, the hollow fiber membrane bundling member is difficult to penetrate between the hollow fiber membranes, and therefore the production is difficult.

$$\phi = \frac{\pi \left( \dfrac{OD}{2} \right)^2 \times n \times N}{\pi \left( \dfrac{ID}{2} \right)^2} \times 100 \qquad \text{(Expression 1)}$$

$\phi$: Filling rate of hollow fiber membrane in module (%)
OD: Outer diameter of hollow fiber membrane (mm)
n: Number of hollow fiber membrane per small bundle (number / bundle)
N: Number of small bundles per module (bundles / module)
ID: Inner diameter of tubular case 3 of module (mm)

A microorganism or a cultured cell used in the production of a chemical substance by continuous fermentation using the present invention will be described.

The microorganism and the cultured cell used in the process for production of a chemical substance using the hollow fiber membrane module of the present invention are not particularly limited. Examples thereof may include yeasts often used in fermentation industry such as a baker's yeast, E. coli, bacteria such as coryneform bacteria, filamentous fungi, actinomycetes, animal cells, insect cells, and the like. The microorganism and the cultured cell to be used may be those which are isolated from natural environment or those in which property thereof is partially modified by mutation or genetic transformation.

A chemical substance obtained by the production process of the present invention, or a converted substance, is a substance produced in a fermentation liquid by the microorganism and the cultured cell. Examples of the chemical substance may include substances produced in large amounts in the fermentation industry, such as alcohols, organic acids, amino acids, and nucleic acids. The present invention can be applied to the production of substances such as enzymes, antibiotics, and recombinant proteins. Examples of the alcohols may include ethanol, 1,3-butanediol, 1,4-butanediol, glycerol, and the like. Examples of the organic acids may include acetic acid, lactic acid, pyruvic acid, succinic acid, malic acid, itaconic acid, citric acid, and the like. Examples of the nucleic acids may include inosine, guanosine, cytidine, and the like.

It is preferable that the converted substance obtained by the production process of the present invention be a fluid containing at least one kind among chemical products, dairy products, pharmaceuticals, foods, and brewing products, or drainage. Herein, examples of the chemical products may include substances applicable to production of chemicals by a step after membrane separation filtration, such as organic acids, amino acids, and nucleic acids. Examples of the dairy products may include substances applicable as dairy products by the step after membrane separation filtration, such as low fat milk. Examples of the pharmaceuticals may include substances applicable to production of pharmaceuticals by the step after membrane separation filtration such as enzymes, antibiotics, and recombinant proteins. Examples of the foods may include substances applicable as foods by the step after membrane separation filtration, such as a lactic acid drink. Examples of the brewing products may include substances applicable as a beverage containing alcohol by the step after membrane separation filtration, such as beer and distilled spirit. Examples of the drainage may include drainage after cleaning products such as food cleaning drainage and dairy product cleaning drainage, domestic drainage rich in the organic materials, and the like.

When lactic acid is produced by the present invention, it is preferable that a yeast be used in the case of an eukaryotic cell and a lactic acid bacterium be used in the case of a prokaryotic cell. Among them, the yeast is preferably yeast obtained by introducing a gene coding lactate dehydrogenase into a cell. It is suitable that as the lactic acid bacterium, a lactic acid bacterium which produces lactic acid having a yield to consumed glucose of 50% or more, and more preferably 80% or more be used.

Examples of the lactic acid bacterium preferably used in the production of lactic acid by the present invention may include, as a wild-type strain, bacteria belonging to Genus Lactobacillus, Genus Bacillus, Genus Pediococcus, Genus Tetragenococcus, Genus Carnobacterium, Genus Carnobacterium, Genus Carnobacterium, Genus Carnobacterium, Genus Vagococcus, Genus Leuconostoc, Genus Oenococcus, Genus Atopobium, Genus Streptococcus, Genus Enterococcus, Genus Lactococcus, and Genus Sporolactobacillus, which have a function synthesizing a lactic acid.

Further, the lactic acid bacteria having high yield of lactic acid to saccharide and high optical purity can be selected and used, and examples of a lactic acid bacterium having a function of selecting and producing D-lactic acid may include a D-lactic acid production bacterium belonging to Genus Sporolactobacillus. As a preferable specific example, Sporolactobacillus laevolacticus or Sporolactobacillus inulinus can be used. More preferable examples thereof may include Sporolactobacillus laevolacticus ATCC23492, ATCC23493, ATCC23494, ATCC23495, ATCC23496, ATCC223549, IAM12326, IAM12327, 1AM12328, IAM12329, IAM12330, IAM12331, IAM12379, DSM2315, DSM6477, DSM6510, DSM6511, DSM6763, DSM6764, and DSM6771, and Sporolactobacillus inulinus JCM6014.

Examples of a lactic acid bacterium having high yield of L-lactic acid to saccharide may include Lactobacillus yamanashiensis, Lactobacillus animalis, Lactobacllus agilis, Lactabacillus aviaries, Lactobacus casei, Lactobacillus delbruekii, Lactobacillus paracasei, Lactobacillus rhamnosus, Lactobacillus ruminis, Lactobacillus salivarius, Lactobacillus sharpeae, Pediocoecus dextrinicus, Lactococcus lactis, and the like. They can be selected and used for the production of L-lactic acid.

In the present invention, a transmembrane pressure difference during filtration of fermentation liquid of microorganisms or cultured cells through a separation membrane in the membrane module may satisfy a condition in which the membrane module does not easily clog with the microorganisms or the cultured cells and medium components. Filtration in a range of transmembrane pressure difference of 0.1 kPa or more and 20 kPa or less is important. The transmembrane pressure difference preferably falls within a range of 0.1 kPa or more and 10 kPa or less, and more preferably a range of 0.1 kPa or more to 5 kPa. When the transmembrane pressure difference is outside the range, the clogging with prokaryotic microorganisms and medium components rapidly occurs, the permeated water reduces, and any problem is sometimes caused in a continuous fermentation operation.

The transmembrane pressure difference can occur in the separation membrane using a siphon utilizing the difference of liquid level (head of water surface) between fermentation liquid and porous membrane treated water or a cross flow circulating pump as a driving force of the filtration. Further, a suction pump may be provided on a separation membrane treated water side as the driving force of the filtration. When the cross flow circulating pump is used, the transmembrane pressure difference can be controlled by a suction pressure. Further, the transmembrane pressure difference can be controlled by a pressure of a gas or liquid introducing a pressure on a fermentation liquid side. When such pressure control can be performed, a difference between a pressure on the fermentation liquid side and a pressure on the porous membrane treated water side is regarded as a transmembrane pressure difference, and can be used for control of the transmembrane pressure difference.

A fermentation raw material is used for the production of a chemical substance by continuous fermentation using the hollow fiber membrane module of the present invention. The fermentation raw material to be used is not limited as long as the growth of microorganisms to be cultured can be promoted and a chemical substance as a fermentation product of interest can be produced well.

The fermentation raw material to be used may be a typical liquid medium appropriately containing a carbon source, a nitrogen source, mineral salts, and if needed, organic micronutrients such as amino acids and vitamins. As the carbon source, saccharides such as glucose, sucrose, fructose, galactose, and lactose, a starch saccharified solution containing these saccharides, sweet potato molasses, beet molasses, high test molasses, organic acids such as acetic acid, alcohols such as ethanol, glycerin, or the like is used. As the nitrogen source, ammonia gas, ammonia water, ammonium salts, urea, nitrates, another organic nitrogen source used adjunctively such as oil cakes, soy hydrolysate liquid, casein decomposition product, another amino acid, vitamins, corn steep liquor, yeast, yeast extract, meat extract, peptides such as peptone, various fermentation bacterial cells and hydrolysates thereof, or the like is used. As the mineral salts, phosphate, a magnesium salt, a calcium salt, an iron salt, a manganese salt, or the like can be appropriately added.

When a particular nutrient is required for the growth of the microorganisms or the cultured cells used for the production of a chemical substance by continuous fermentation, the nutrient is added as an authentic sample or a natural product containing it. An antifoamer can be used, if needed. In the production of a chemical substance by continuous fermentation, a broth is referred to as a liquid obtained after proliferation of microorganisms or cultured cells in a fermentation raw material. The composition of additional fermentation raw material may be appropriately altered from the composition of a fermentation raw material at the start of culture so as to increase the productivity of target chemical substance.

In the production of a chemical substance by continuous fermentation, the concentration of saccharides in the fermentation broth is preferably maintained to 5 g/l or less. Preferable reason in which the concentration of saccharides in the fermentation broth is maintained to 5 g/l or less is to minimize the loss of saccharides due to drawing of the fermentation broth.

The culture of microorganisms or cultured cells is usually performed within a range of a pH of 4 or more and 8 or less and a temperature of 20°C or higher and 40°C or lower. The pH of the fermentation broth is usually adjusted to a predetermined value of a pH of 4 or more and 8 or less by an inorganic acid, an organic acid, an alkaline substance, urea, calcium carbonate, ammonia gas, or the like. When increase of a rate of feed of oxygen is required, maintenance of the oxygen concentration to 21% or more by addition of oxygen to air, pressurization of a fermentation broth, increase of a stirring rate, increase of airflow amount, or the like can be used as a means for increasing the rate.

In the production of a chemical substance by continuous fermentation, since counter-pressure cleaning or cleaning due to immersion in chemicals is used for the cleaning of a separation membrane, durability to these is required. For example, water or a filtered liquid is used as a counter-pressure cleaning liquid. Further, in a range in which the fermentation is not largely inhibited, an alkali, an acid, or an oxidizing agent can be used. Examples of the alkali may include a sodium hydroxide aqueous solution, a calcium hydroxide aqueous solution, and the like. Examples of the acid may include oxalic acid, citric acid, hydrochloric acid, nitric acid, and the like. Examples of the oxidizing agent may include a hypochlorite aqueous solution, hydrogen peroxide solution, and the like. A liquid for counter-pressure cleaning can be used at a high temperature less than 100°C. As used herein, the counter-pressure cleaning is a method for removing dirt matters on a membrane surface by sending the liquid from a filtered liquid side as a secondary side of a separation membrane to a fermentation liquid side as a primary side.

Therefore, in the hollow fiber membrane module of the present invention, durability to a pH of 2 to 12, an alkali, an acid, or an oxidizing agent, and water at high temperature is usually required in addition to the durability to the steam sterilization.

The counter-pressure cleaning rate of counter-pressure cleaning liquid falls within a range of 0.5 or more and 5 or less times, and more preferably a range of 1 or more and 3 or less times as fast as the membrane filtration rate. When the counter-pressure cleaning rate exceeds this range, the separation membrane may be damaged. When it is lower than this range, the cleaning effect may not be sufficiently achieved.

The counter-pressure cleaning cycle of counter-pressure cleaning liquid can be determined depending on a transmembrane pressure difference and a variation of the transmembrane pressure difference. The counter-pressure cleaning cycle per hour falls within a range of 0.5 or more and 12 or less, and more preferably 1 or more and 6 or less. When the counter-pressure cleaning cycle exceeds this range, the separation membrane may be damaged. When it is less than this range, the cleaning effect may not be sufficiently obtained.

The counter-pressure cleaning time of counter-pressure cleaning liquid can be determined depending on a counter-pressure cleaning cycle, a transmembrane pressure difference, and a variation of the transmembrane pressure difference. The counter-pressure cleaning time per cycle falls within a range of 5 seconds or more and 300 seconds or less, and more preferably 30 seconds or more and 120 seconds or less. When the counter-pressure cleaning time is longer than this range, the separation membrane may be damaged. When it is shorter than this range, the cleaning effect may not be sufficiently obtained.

Further, during the counter-pressure cleaning, filtration is once stopped, a separation membrane can be immersed in a counter-pressure cleaning liquid. The immersion time can be determined depending on an immersion cleaning cycle, a transmembrane pressure difference, and a variation of the transmembrane pressure difference. The immersion time per step preferably falls within a range of 1 minute or more and 24 hours or less, and more preferably 10 minutes or more and 12 hours or less.

When a multiple system is used in the separation membrane, the system is exchanged during cleaning by immersion of the separation membrane in a counter-pressure cleaning liquid not to stop the whole filtration. This can be preferably utilized.

In the production of a chemical substance by continuous fermentation, Batch culture or Fed-batch culture is performed in an early stage of the culture to increase the microorganism concentration, and the continuous fermentation (drawing) may be started. Alternatively, the microorganism concentration increases, microorganism in a high concentration are then seeded, and the continuous fermentation may be performed during initiation of the culture. In the production of a chemical substance by continuous fermentation, a raw material broth can be supplied and a cultured substance can be drawn from the suitable period. The initiation period of supply of raw material broth and that of drawing of cultured substance are not necessarily the same. Further, the supply of raw material broth and the drawing of the cultured substance may be continuous or intermittent.

Nutrients necessary for the proliferation of bacterial cells may be added to the raw material broth to continuously proliferate the bacterial cells. As a preferable aspect to obtain effective productivity, the concentration of microorganisms or cultured cells in the fermentation broth is kept high within a range not increasing a ratio in which the environment of the fermentation broth is not appropriate for the proliferation microorganisms or cultured cells and the cells are killed. As one example of the concentration of the microorganisms or cultured cells in the fermentation broth, the microorganism concentration is kept at 5 g/L or more as a dry weight in D-lactic acid fermentation using SL lactic acid bacteria to obtain good production efficiency.

In the production of a chemical substance by continuous fermentation, the microorganisms or cultured cells can be drawn from the inside of a fermenter, if necessary. For example, when the concentration of the microorganisms or cultured cells in the fermenter increases, the closing in the separation membrane is likely to occur. Therefore, by drawing, the clogging can be avoided. Further, the production performance of a chemical substance is sometimes varied by the concentration of the microorganisms or cultured cells in the fermenter, but the production performance can be maintained by drawing the microorganisms or cultured cells using the production performance as an indication.

In the production of a chemical substance by continuous fermentation, the number of fermentation reaction tank is not limited as long as a continuous culture operation performed with proliferation of fresh bacterial cells having a fermentation production capacity is a continuous culture process in which bacterial cells are proliferated and substantially products are produced. In the production of a chemical substance by continuous fermentation, it is preferable that the continuous culture operation be typically performed in a single fermentation reaction tank for culture control. A plurality of fermentation reaction tanks may be used when the capacity of the fermentation reaction tank is small. In this case, the plurality of fermentation reaction tanks are disposed and connected in parallel or series and the continuous culture is performed, and thus high productivity of fermentation product is obtained.

Examples

In order to describe effects of the present invention in detail below, a specific embodiment of continuous fermentation using an apparatus shown in the schematic view of FIG. 4 by a microorganism having an ability of selecting D-lactic acid as the chemical substance and producing D-lactic acid will be described with reference of Examples.

(Reference Example 1) Production of hollow fiber membrane

A vinylidene fluoride homopolymer having a weight average molecular weight of 417,000 and γ-butyrolactone were dissolved at a proportion of 38% by weight and 62% by weight, respectively, at a temperature of 170°C. The macromolecular solution was discharged from a sleeve with γ-butyrolactone as a hollow section-forming fluid, and solidified in a cooling bath of a solution of 80% by weight of γ-butyrolactone at a temperature of 20°C to produce a hollow fiber membrane of a spherical structure. 14% by weight of vinylidene fluoride homopolymer having a weight average molecular weight of 284,000, 1% by weight of cellulose acetate propionate (available from Eastman Chemical Company, CAP482-0.5), 77% by weight of N-methyl-2-pyrrolidone, 5% by weight of T-20C, and 3% by weight of water were mixed and dissolved at this proportion at a temperature of 95°C to prepare a macromolecular solution. The raw material for membrane production was applied uniformly to the surface of the hollow fiber membrane of a spherical structure, and immediately solidified in a water bath to produce a hollow fiber membrane having a three-dimensional network structure formed on a spherical structure layer. The average micropore diameter of a surface on a water-treated side of the obtained hollow fiber membrane was 0.04 $\mu$m. When a pure water permeation volume was evaluated on the hollow fiber porous membrane as a separation membrane, it was $5.5 \times 10^{-9}$ m$^3$/m$^2$/s/Pa. The measurement of the water permeation volume at a head height of 1 m was performed using purified water at a temperature of 25°C obtained by reverse osmosis membrane filtration.

(Example 1)

A hollow fiber membrane module was produced using a molded product which was a tubular container made of a polysulfone resin as a separation membrane module case. A configuration in which a nozzle capable of introducing a fermentation broth into a lower side part of the module case was provided was used as shown in FIG. 1. The hollow fiber membrane produced in Reference Example 1 was used as a separation membrane and was brought into contact with saturated steam at 121°C for 1 hour. In the contact with saturated steam, an autoclave "LSX-700" manufactured by TOMY SEIKO CO., LTD., was used. The hollow fiber membrane was inserted into the module case, both ends of the hollow fiber membrane were adhered to the module case using a urethane resin (available from SANYU REC CO., LTD., two chemicals SA-7068A and SA-7068B were mixed so that a weight ratio was 64:100). In an upper end of the module, an excess adhering part was cut and used so as to open the hollow fiber membrane.

The hollow fiber membrane module produced as described above was used to perform continuous culture. The filling rate of the hollow fiber membrane in the module was 45%. A fermentation broth was introduced from the nozzle at the lower side part of the module and was returned to a fermenter 12 from the nozzle at the lower side part of the module.

In order to measure a hardness retention of an urethane resin used for a hollow fiber membrane bundling member 4, 30 g of the urethane resin was sufficiently mixed by stirring, and hardened at 80°C for 4 hours so as to become a plate having a thickness of 6 mm. The resin stood at room temperature, and was naturally cooled to 25°C. After then, measurement was performed with a type D durometer according to JIS-K6253 by pushing a probe into the surface of the resin. The resin stood at room temperature, and was naturally cooled to 25°C, and a D hardness was measured with the type D durometer according to JIS-K6253. A type D durometer hardness according to JIS-K6253 (2004) was measured using a measuring device according to JIS-K6253(2004) by pushing a probe into the surface of the resin. The resin of which the hardness had been measured was brought into contact with saturated steam at 121°C for 24 hours, and naturally cooled to 25°C. Moisture on the surface was cleanly removed. The hardness was then measured by the above-described method. In the contact with saturated steam, the autoclave "LSX-700" manufactured by TOMY SEIKO CO., LTD., was used. As a result, the hardness of the urethane resin used for the hollow fiber membrane bundling member 4 was 55, and the hardness retention of the hardened material under a saturated steam condition of 121°C and 2 atmospheric pressure after 24 hours was 97%.

D-lactic acid was prepared using the hollow fiber membrane module produced as described above, the continuous fermentation apparatus of FIG. 4, and a lactic acid fermentation medium having a composition shown in Table 1. The medium was subjected to steam sterilization under saturated steam at 121°C for 20 minutes and used. In high-pressure steam sterilization, the autoclave "LSX-700" manufactured by TOMY SEIKO CO., LTD., was used. A hollow fiber membrane module 1 was connected with a fermenter 12, and was subjected to steam sterilization under saturated steam at 121°C for 20 minutes before use. The steam sterilization of the hollow fiber membrane module 1 under saturated steam at 121°C for 20 minutes was repeated 20 times, and there was no problem of leakage and the like.

Operation conditions were as follows:

Volume of fermenter: 2 L

Effective volume of fermenter: 1.5 L

Separation membrane used: 60 polyvinylidene fluoride hollow fiber membranes (effective length: 8 cm, total effective membrane area: 0.020 m$^2$)

Temperature adjustment: 37°C

Airflow volume of fermenter: nitrogen gas 0.2 L/min

Stirring speed of fermenter: 600 rpm

pH adjustment: adjustment of pH to 6 by 3N Ca(OH)$_2$

Supply of lactic acid fermentation medium: adding so as to control the volume of liquid in a fermenter to a constant volume of about 1.5 L

Volume of liquid circulated by fermentation liquid circulating apparatus: 2 L/min

Control of membrane filtration flow rate: control of flow rate by a suction pump

Intermittent filtration: Operation at a cycle of filtration (9 minutes) and filtration stop processing (1 minute) Membrane filtration flux: variable so that the transmembrane pressure difference is 20 kPa or less in a range of 0.01 m/day or more and 0.3 m/day or less. When the transmembrane pressure difference exceeds the range and was continued to increase, the continuous fermentation was completed.

The medium was subjected to steam sterilization under saturated steam at 121°C for 20 minutes and used. Sporolactobacillus laevolacticus JCM2513 (SL strain) was used as a microorganism, and a lactic acid fermentation medium having a composition shown in Table 1 was used as a medium. The concentration of lactic acid as a product was evaluated under the following conditions using HPLC shown below.

Table 1
Lactic Acid Fermentation Medium

| Component | Amount |
|---|---|
| Glucose | 100 g |
| Yeast Nitrogen base W/O amino acid (Difco Laboratories Inc.) | 6.7 g |

(continued)

Lactic Acid Fermentation Medium

| Component | Amount |
|---|---|
| 19 Kinds of Standard Amino Acids Excluding Leucine | 152 mg |
| Leucine | 760 mg |
| Inositol | 152 mg |
| p-Aminobenzoic Acid | 16 mg |
| Adenine | 40 mg |
| Uracil | 152 mg |
| Water | 892 g |

Column: Shim-Pack SPR-H (manufactured by Shimadzu Corporation)
Mobile phase: 5 mM p-toluenesulfonic acid (0.8 mL/min)
Reaction phase: 5 mM p-toluenesulfonic acid, 20 mM bis-tris, 0.1 mM EDTA 2Na (0.8 mL/min)
Detection method: electric conductivity
Column temperature: 45°C

The optical purity of lactic acid was analyzed under the following conditions.

Column: TSK-gel Enantio L1 (manufactured by TOSOH CORPORATION)
Mobile phase: 1 mM copper sulfate aqueous solution
Flow rate: 1.0 mL/min
Detection method: UV 254 nm
Temperature: 30°C

The optical purity of L-lactic acid was calculated by the following expression (i).

$$\text{Optical purity (\%)} = 100 \times (L - D)/(D + L) \qquad \text{(i)}$$

The optical purity of D-lactic acid was calculated by the following expression (ii).

$$\text{Optical purity (\%)} = 100 \times (D - L)/(D + L) \qquad \text{(ii)}$$

In the expressions, L represents the concentration of L-lactic acid and D represents the concentration of D-lactic acid.
An SL strain was subjected to shake culture in a 5-mL lactic acid fermentation medium of a test tube overnight (pre-pre-preculture). The obtained broth was inoculated to 100 mL of fresh lactic acid fermentation medium, and was subjected to shake culture in a 500-mL sakaguchi flask at 30°C for 24 hours (pre-preculture). A broth of the pre-preculture was placed in a 1.5-L fermenter of the continuous fermentation apparatus shown in FIG. 1 and inoculated, and was stirred in a fermenter 12 using an accessory stirrer 16. The adjustment of airflow volume, temperature, and pH of the fermenter 12 was performed, and 24-hour culture was performed without a fermentation broth circulating pump 14 driven (preculture). Immediately after completion of the preculture, the fermentation broth circulating pump 14 was driven, a lactic acid fermentation medium was continuously supplied in addition to the operation conditions during the preculture. The continuous fermentation was performed with the membrane water permeation volume controlled so that the fermentation liquid volume in the continuous fermentation apparatus was 2 L to produce D-lactic acid by continuous fermentation. The membrane water permeation volume during a continuous fermentation test was controlled so that the filtration volume was the same as the supply volume of the fermentation medium by a metering filtration pump 13. The concentrations of D-lactic acid produced in a fermentation broth which had permeated the membrane and of remaining glucose were appropriately measured.
The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 380-hour continuous fermentation was able to be performed, and the fastest rate of production of D-lactic acid was 2.1 [g/L/hr].

Table 2

| | Example 1 | Example 2 | Example 3 | Example 4 | Example 5 | Example 6 | Example 7 |
|---|---|---|---|---|---|---|---|
| Fermentation Time [hr] | 380 | 430 | 350 | 400 | 500 | 350 | 250 |
| Fastest D-lactic acid production rate [g/Uhr] | 2.1 | 2.3 | 1.9 | 2.1 | 2.6 | 1.7 | 0.9 |

(Example 2)

A hollow fiber membrane module was produced using a molded product which was a tubular container made of a polysulfone resin in a configuration shown in FIG. 2, as a separation membrane module case. The hollow fiber membrane produced in Reference Example 1 was used as a separation membrane, and was cut into a length twice the whole length of the module case, and brought into contact with saturated steam at 121°C for 1 hour. The sterilized hollow fiber membrane was folded in half, and a folded part of the hollow fiber was inserted in a cap made of a polysulfone resin to which an urethane resin was injected (available from SANYU REC CO., LTD., two chemicals SA-7068A and SA-7068B were mixed so that a weight ratio was 64:100) to form 7 small bundles. At this time, the number of hollow fiber membrane per small bundle was 750 and the filling rate of the hollow fiber membrane in the module was 45%. The small bundles dried over 24 hours were inserted in the module case, and the module case adhered to each small bundle using the urethane resin (available from SANYU REC CO., LTD., two chemicals SA-7068A and SA-7068B were mixed so that a weight ratio was 64:100). In an upper part of the module, an excess adhering part was cut and used so as to open the hollow fiber membrane.

The continuous culture was performed in the same manner as in Example 1 using the hollow fiber membrane module produced as described above. The filling rate of the hollow fiber membrane in the module was 45%. A fermentation broth was introduced from a nozzle at the lower part of the module and was returned to a fermenter 12 from a nozzle at an upper side part of the module.

In order to measure a hardness retention of an urethane resin used for a hollow fiber membrane bundling member 4, the D hardness was measured in the same manner as in Example 1. As a result, the hardness of the urethane resin used for the hollow fiber membrane bundling member 4 was 61, and the hardness retention of the hardened material under a saturated steam condition of 121°C and 2 atmospheric pressure after 24 hours was 99%.

D-lactic acid was produced in the same manner as in Example 1 using the hollow fiber membrane module produced as described above, the continuous fermentation apparatus of FIG. 4, and the lactic acid fermentation medium having a composition shown in Table 1. The medium was subjected to steam sterilization under saturated steam at 121°C for 20 minutes and used. The hollow fiber membrane module 1 was connected with the fermenter 12, and was subjected to steam sterilization under saturated steam at 121°C for 20 minutes before use. The steam sterilization of the hollow fiber membrane module 1 under saturated steam at 121°C for 20 minutes was repeated 20 times, and there was no problem of leakage and the like.

The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 430-hour continuous fermentation was able to be performed, and the fastest rate of production of D-lactic acid was 2.3 [g/L/hr].

(Example 3)

The hollow fiber membrane produced in Reference Example 1 was used as a separation membrane and was brought into contact with saturated steam at 105°C for 1 hour. The hollow fiber membrane module was produced and the continuous culture was performed in the same manner as in Example 2.

The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 350-hour continuous fermentation was able to be performed, and the fastest rate of production of D-lactic acid was 1.9 [g/L/hr].

(Example 4)

The hollow fiber membrane produced in Reference Example 1 was used as a separation membrane and was brought into contact with saturated steam at 110°C for 1 hour. The hollow fiber membrane module was produced and the continuous culture was performed in the same manner as in Example 2.

The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 400-hour continuous fermentation was able to be performed, and the fastest rate of production of D-lactic acid was 2.1 [g/L/hr].

(Example 5)

The hollow fiber membrane produced in Reference Example 1 was used as a separation membrane and was brought into contact with saturated steam at 130°C for 1 hour. The hollow fiber membrane module was produced and the continuous culture was performed in the same manner as in Example 2.

The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 500-hour continuous fermentation was able to be performed, and the fastest rate of production of D-lactic acid was 2.6 [g/L/hr].

(Example 6)

The hollow fiber membrane produced in Reference Example 1 was used as a separation membrane and was brought into contact with saturated steam at 140°C for 1 hour. The hollow fiber membrane module was produced and the continuous culture was performed in the same manner as in Example 2.

The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 350-hour continuous fermentation was able to be performed, and the fastest rate of production of D-lactic acid was 1.7 [g/L/hr].

(Example 7)

The hollow fiber membrane produced in Reference Example 1 was used as a separation membrane and was brought into contact with saturated steam at 150°C for 1 hour. The hollow fiber membrane module was produced and the continuous culture was performed in the same manner as in Example 2.

The results in the continuous fermentation test are shown in Table 2. In the continuous fermentation apparatus shown in FIG. 4, the hollow fiber membrane module of the present invention was introduced to produce a chemical substance, and thus stable production of D-lactic acid by continuous fermentation was possible. 250-hour continuous fermentation was able to be performed, and the fastest rate of production of D-lactic acid was 0.9 [g/L/hr].

(Comparative Example 1)

A module was produced in the same manner as in Example 1 except that an urethane resin (available from SANYU REC CO., LTD., two chemicals SA-7238A and SA-7238B were mixed so that a weight ratio was 45:100) was used for a hollow fiber membrane bundling member 4 and a small bundle plugging member 5 as a separation membrane module. The hardness of the urethane resin used for the hollow fiber membrane bundling member 4 and the small bundle plugging member 5 was 57 and the hardness retention after contact with saturated steam at 121°C for 24 hours was 56%. D-lactic acid was produced under the same conditions as in Example 1 using the separation membrane module.

The hardness retention of the produced hollow fiber membrane module was low. The steam sterilization of the module under a saturated steam condition of 121°C for 20 minutes was repeated. After a fifth treatment, peeling between the hollow fiber membrane and the urethane resin which was a hollow fiber membrane clogging member was confirmed. In an air leak test at 30 kPa, air leak occurred.

Industrial Applicability

Use of the hollow fiber membrane module of the present invention stably keeps high productivity under an easy operation condition over a long period of time and enables continuous fermentation capable of sterilization treatment. Further, a chemical substance which is a fermentation product can be stably produced at low cost widely in the fermentation industry.

Reference Signs List

| | |
|---|---|
| 1 | HOLLOW FIBER MEMBRANE MODULE |
| 2 | HOLLOW FIBER MEMBRANE |
| 2a | SMALL BUNDLE |
| 3 | TUBULAR CASE |
| 4 | HOLLOW FIBER MEMBRANE BUNDLING MEMBER |
| 5 | SMALL BUNDLE PLUGGING MEMBER |
| 6 | UPPER CAP |
| 7 | LOWER CAP |
| 8 | FERMENTATION BROTH INLET |
| 9 | FILTERED LIQUID OUTLET |
| 10 | FERMENTATION BROTH OUTLET |
| 11 | SMALL BUNDLE CONTAINER |
| 12 | FERMENTER |
| 13 | FILTRATION PUMP |
| 14 | FERMENTATION BROTH CIRCULATING PUMP |
| 15 | GAS SUPPLYING DEVICE |
| 16 | STIRRER |
| 17 | BROTH SUPPLYING PUMP |
| 18 | pH ADJUSTING LIQUID SUPPLYING PUMP |
| 19 | pH SENSOR/CONTROLLER |
| 20 | TEMPERATURE ADJUSTING DEVICE |

**Claims**

1. A hollow fiber membrane module for use in production of a chemical substance, which is used in continuous fermentation including filtering a fermentation broth of a microorganism or a cultured cell through a hollow fiber membrane, collecting a chemical substance from a filtrate, retaining a concentrated solution in the fermentation broth or refluxing the concentrated solution, and adding a fermentation raw material to the fermentation broth, wherein a large number of hollow fiber membrane bundles are accommodated in a tubular case, at least one end part of each of the hollow fiber membrane bundles is fixed on the tubular case by a hollow fiber membrane bundling member with an end face of each of the hollow fiber membranes open, and the hollow fiber membrane bundling member is made of a synthetic resin having a hardness retention rate after contact with saturated steam at 121°C for 24 hours of 95% or more.

2. The hollow fiber membrane module for use in the production of a chemical substance according to claim 1, wherein one end part of each of the hollow fiber membrane bundles is fixed on the tubular case by the hollow fiber membrane bundling member with the end face of each of the hollow fiber membrane open, the other end part of each of the hollow fiber membrane bundles is divided into a plurality of small bundles, and the end face of each of the hollow fiber membranes by the small bundle is plugged by a small bundle plugging member.

3. The hollow fiber membrane module for use in the production of a chemical substance according to claim 1 or 2, wherein the hollow fiber membrane is obtained by bringing a hollow fiber membrane containing a fluororesin-based macromolecule into contact with saturated steam at 110°C or higher and 135°C or lower.

4. The hollow fiber membrane module for use in the production of a chemical substance according to claim 1 or 2, wherein the hollow fiber membrane is obtained by bringing a hollow fiber membrane containing a fluororesin-based macromolecule into contact with saturated steam at 120°C or higher and 130°C or lower.

5. The hollow fiber membrane module for use in the production of a chemical substance according to any of claims 1 to 4, wherein the hollow fiber membrane contains a polyvinylidene fluoride-based resin.

**6.** The hollow fiber membrane module for use in the production of a chemical substance according to any of claims 1 to 5, wherein the hollow fiber membrane contains a hydrophilic macromolecule having at least one kind selected from a fatty acid vinyl ester, vinyl pyrrolidone, ethylene oxide, and propion oxide, or a cellulose ester.

**7.** A process for production of a chemical substance using the hollow fiber membrane module for use in the production of a chemical substance according to any of claims 1 to 6.

# FIG.1

# FIG.2

# FIG.3

# FIG.4

<div align="center">

**INTERNATIONAL SEARCH REPORT**

</div>

| | International application No. |
|---|---|
| | PCT/JP2010/069969 |

A. CLASSIFICATION OF SUBJECT MATTER
*B01D63/04*(2006.01)i, *B01D63/00*(2006.01)i, *B01D71/34*(2006.01)i, *C12M1/12* (2006.01)n, *C12M3/06*(2006.01)n, *C12N15/09*(2006.01)n, *C12P7/56*(2006.01)n

According to International Patent Classification (IPC) or to both national classification and IPC

B. FIELDS SEARCHED

Minimum documentation searched (classification system followed by classification symbols)
B01D63/04, B01D63/00, B01D71/34, C12M1/12, C12M3/06, C12N15/09, C12P7/56

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-237101 A (Toray Industries, Inc.), 09 October 2008 (09.10.2008), particularly, claims; paragraphs [0100], [0101] (Family: none) | 1-7 |
| A | JP 2008-99667 A (Toray Industries, Inc.), 01 May 2008 (01.05.2008), particularly, examples (Family: none) | 1-7 |
| P,A | JP 2010-29108 A (Toray Industries, Inc.), 12 February 2010 (12.02.2010), particularly, claims; examples (Family: none) | 1-7 |

| ☐ Further documents are listed in the continuation of Box C. | ☐ See patent family annex. |
|---|---|

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 07 February, 2011 (07.02.11) | 22 February, 2011 (22.02.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007252367 A
- JP 2008237101 A
- JP H0760074 B
- JP 2005230813 A

**Non-patent literature cited in the description**

- **TOSHIHIKO HIRAO.** *Appl. Microbiol. Biotechnol.,* 1989, vol. 32, 269-273